(19) Europäisches Patentamt
European Patent Office
Office européen des brevets

(11) Veröffentlichungsnummer: **0 394 829**
**A1**

(12) # EUROPÄISCHE PATENTANMELDUNG

(21) Anmeldenummer: **90107395.7**

(22) Anmeldetag: **19.04.90**

(51) Int. Cl.[5]: **C07K 5/06, C07K 5/02, A61K 37/64**

(30) Priorität: **22.04.89 DE 3913290**

(43) Veröffentlichungstag der Anmeldung: **31.10.90 Patentblatt 90/44**

(84) Benannte Vertragsstaaten: **AT BE CH DE DK ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Wagner, Adalbert, Dr.**
**Kleiststrasse 9**
**D-6238 Hofheim am Taunus(DE)**
Erfinder: **Urbach, Hansjörg, Dr.**
**Le Lavandoustrasse 41**
**D-6242 Kronberg/Taunus(DE)**
Erfinder: **Ruppert, Dieter, Dr.**
**Schreyerstrasse 30**
**D-6242 Kelheim (Taunus)(DE)**
Erfinder: **Linz, Wolfgang, Dr.**
**Gundelhardtstrasse 2**
**D-6233 Kelkheim (Taunus)(DE)**
Erfinder: **Schölkens, Bernward, Dr., Prof.**
**Hölderlinstrasse 62**
**D-6233 Kelkheim (Taunus)(DE)**

(54) **Renin-hemmende Dipeptide, Verfahren zu deren Herstellung, diese enthaltende Mittel und ihre Verwendung.**

(57) Die vorliegende Erfindung betrifft Verbindungen der Formel I

$$R^1-A-B-HN-\underset{\displaystyle\phantom{|}}{\overset{\displaystyle R^2}{\overset{|}{C}}}H-\overset{\displaystyle OH}{\overset{|}{C}}H-\overset{\displaystyle R^3}{\overset{|}{C}}H-R^4 \qquad (I)$$

in welcher
A und B unabhängig voneinander eine Aminosäure stehen und $R^1$, $R^2$ $R^3$ und $R^4$ wie in der Beschreibung angegeben definiert sind,
Verfahren zu ihrer Herstellung, ihre Verwendung als Heilmittel und iese enthaltende pharmazeutische Mittel.

EP 0 394 829 A1

# Renin-hemmende Dipeptide, Verfahren zu deren Herstellung, diese enthaltende Mittel und ihre Verwendung

Aus den EP-A-152 255, EP-A-155 809, EP-A-163 237, EP-A-172 346, EP-A-172 347, EP-A-179 352 und EP-A-255 082 sind Di- und Tripeptidderivate und deren Verwendung als Renin-Inhibitoren bekannt.

Es wurden neue Peptidderivate gefunden, die in vitro und in vivo hochwirksam das Enzym Renin hemmen.

Die Erfindung betrifft Verbindungen der Formel I

$$R^1\text{-A-B-HN-}\overset{\displaystyle R^2}{\overset{|}{C}}\text{H-}\overset{\displaystyle OH}{\overset{|}{C}}\text{H-}\overset{\displaystyle R^3}{\overset{|}{C}}\text{H-}R^4 \qquad (I)$$

in welcher

$R^1$ einen Rest der Formel II bedeutet,

$R^a$-W- (II)

worin W für -CO-, -O-CO-, $-SO_2-$ oder -NH-CO- steht und

$R^a$ Wasserstoff, $(C_1-C_{10})$-Alkyl, das gegebenenfalls ein-oder zweifach ungesättigt ist und das gegebenenfalls durch bis zu 3 gleiche oder verschiedene Reste aus der Reihe Hydroxy, $(C_1-C_7)$-Alkoxy, $(C_1-C_7)$-Alkanoyloxy, Carboxy, $(C_1-C_7)$-Alkoxycarbonyl, Halogen, Amino, $(C_1-C_7)$-Alkylamino, Di-$(C_1-C_7)$-alkylamino, $(C_1-C_5)$-Alkoxycarbonylamino, $(C_7-C_{15})$-Aralkoxycarbonylamino und 9-Fluorenylmethyloxycarbonylamino substituiert ist, $(C_3-C_8)$-Cycloalkyl, $(C_3-C_8)$-Cycloalkyl-$(C_1-C_6)$-alkyl, $(C_6-C_{14})$-Aryl, das gegebenfalls durch einen oder zwei gleiche oder verschiedene Reste aus der Reihe F, Cl, Br, J, Hydroxy, $(C_1-C_7)$-Alkoxy, $(C_1-C_7)$-Alkyl, $(C_1-C_7)$-Alkoxycarbonyl, Amino, gegebenenfalls mit bis zu 2 Halogen substituiertes Anilino und Trifluormethyl substituiert ist,

$(C_6-C_{14})$-Aryl-$(C_1-C_6)$-alkyl, worin der Arylteil gegebenenenfalls durch einen oder zwei gleiche oder verschiedene Reste aus der Reihe F, Cl, Br, J, Hydroxy, $(C_1-C_7)$-Alkoxy, $(C_1-C_7)$-Alkyl, $(C_1-C_7)$-Alkoxycarbonyl, Amino, $(C_1-C_7)$-Alkylamino, Di-$(C_1-C_7)$-alkylamino, Carboxy, Carboxymethoxy, Amino-$(C_1-C_7)$-alkyl, $(C_1-C_7)$-Alkylamino-$(C_1-C_7)$-alkyl, Di-$(C_1-C_7)$-alkylamino $(C_1-C_7)$-alkyl, $(C_1-C_7)$-Alkoxycarbonylmethoxy, Carbamoyl, Sulfamoyl, $(C_1-C_7)$-Alkoxysulfonyl, Sulfo und Guanidinomethyl substituiert ist, oder für den Rest eines 5- oder 6-gliedrigen monocyclischen oder 9- oder 10-gliedrigen bicyclischen Heterocyclus mit mindestens 1 C-Atom, 1 -4 N-Atomen und/oder 1 S- oder O-Atom auch als Ringglieder steht, der gegebenenfalls durch einen oder mehrere Reste aus der Reihe F, Cl, Br, Hydroxy, $(C_1-C_7)$-Alkoxy, $(C_1-C_7)$-Alkyl, $(C_2-C_8)$-Alkoxycarbonyl, Amino oder Trifluormethyl, di- oder trisubstituiert ist,

$R^4$ einen Rest der Formel III bedeutet,

$-X-(CH_2)_n-R^7$ (III)

A einen N-terminal mit $R^1$ und C-terminal mit B verknüpften Rest einer Aminosäure aus der Reihe Phenylalanin, Histidin, Tyrosin, Tryptophan, Methionin, Leucin, Isoleucin, Asparagin, Asparaginsäure, $\beta$-2-Thienylalanin, $\beta$-3-Thienylalanin, $\beta$-2-Furanylalanin, $\beta$-3-Furanylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, 4-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, $\beta$-2-Benzo(b)thienylalanin, $\beta$-3-Benzo(b)thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyldopa, 2-Amino-4-(2-thienyl)-buttersäure, 2-Amino-4-(3-thienyl)-buttersäure, 3-(2-Thienyl)-serin, (Z)-Dehydrophenylalanin und (E)-Dehydrophenylalanin bedeutet,

B einen Rest einer Aminosäure, wie unter A definiert, bedeutet,

$R^2$ Wasserstoff, $(C_1-C_{10})$-Alkyl, $(C_4-C_7)$-Cycloalkyl, $(C_4-C_7)$-Cycloalkyl-$(C_1-C_4)$-alkyl, $(C_6-C_{14})$-Aryl oder $(C_6-C_{14})$-Aryl-$(C_1-C_4)$-alkyl bedeutet

$R^3$ Wasserstoff, $(C_1-C_{10})$-Alkyl, $(C_6-C_{14})$-Aryl oder $(C_6-C_{14})$-Aryl-$(C_1-C_4)$-alkyl bedeutet,

X wahlweise abwesend sein kann oder O oder S bedeutet,

n = 2, 3 oder 4 sein kann und

$R^7$ Hydroxy oder Amino bedeutet, oder für den Rest eines 5-gliedrigen Heterocyclus mit mindestens 1 C-Atom, 1-4 N-Atomen und/oder 1 S- oder O-Atom, der gegebenenfalls durch einen oder mehrere Reste aus der Reihe F, Cl, Br, Hydroxy, $(C_1-C_7)$-Alkoxy, $(C_1-C_7)$-Alkyl, $(C_2-C_8)$-Alkoxycarbonyl, Amino oder Trifluorme-

thylsubstituiert ist, steht, wobei Oxazolin ausgenommen ist sowie deren physiologisch verträglichen Salze.

Die durch $R^2$, Hydroxy und $R^3$ substituierten C-Atome können jeweils die R-, S- oder R,S-Konfiguration besitzen.

Alkyl kann geradkettig oder verzweigt sein. Entsprechendes gilt für davon abgeleitete Reste, wie z.B. Alkoxy, Alkylthio, Alkylamino, Dialkylamino, Alkanoyl und Aralkyl.

Unter Cycloalkyl werden auch alkylsubstituierte Reste, wie z.B. 4-Methylcyclohexyl oder 2,3-Dimethylcyclopentyl verstanden.

($C_6$-$C_{14}$)-Aryl ist beispielsweise Phenyl, Naphtyl, Biphenylyl oder Fluorenyl; bevorzugt ist Phenyl. Entsprechendes gilt für davon abgeleitete Reste, wie z.B. Aryloxy, Aroyl, Aralkyl und Aralkyloxy. Unter Aralkyl versteht man einen mit ($C_1$-$C_6$)-Alkyl verknüpften unsubstituierten oder substituierten ($C_6$-$C_{14}$)-Aryl-Rest, wie z.B. Benzyl, α- und β-Naphthylmethyl, Halobenzyl und Alkoxybenzyl, wobei Aralkyl jedoch nicht auf die genannten Verbindungen beschränkt wäre.

Unter einem für $R^a$ stehenden Rest eines 5- oder 6-gliedrigen monocyclischen oder 9- oder 10-gliedrigen bicyclischen Heterocyclus mit mindestens 1 C-Atom, 1-4 N-Atomen und/oder 1 S- oder O-Atom als Ringglieder werden Reste von Heterocyclen verstanden, wie sie beispielsweise in Katritzky, Lagowski, Chemie der Heterocyclen, Berlin, Heidelberg 1968, Seite 3 - 5 definiert sind. Monocyclische Heterocyclen sind z.B. Thiophen, Furan, Pyrrol, Imidazol, Pyrazol, Pyridin, Pyrazin, Pyrimidin, Pyridazin, 1,2,4-Triazol, Thiazol, Tetrazol, Isothiazol, Oxazol und Isoxazol sowie die davon abgeleiteten ganz oder teilweise hydrierten Heterocyclen. Bicyclische Heterocyclen sind z.B. Benzothiophen, Benzofuran, Indol, Isoindol, Indazol, Benzimidazol, Chinolin, Isochinolin, Phthalazin, Chinoxalin, Chinazolin und Cinnolin sowie die davon abgeleiteten ganz oder teilweise hydrierten Heterocyclen.

Unter einem für $R^7$ stehenden Rest eines 5-gliedrigen Heterocyclus mit mindestens 1 C-Atom. 1-4 N-Atomen und/oder 1 S- oder O-Atom werden insbesondere Thiophen, Furan, Pyrrol, Imidazol, Pyrazol, 1.2.4-Triazol, Thiazol, Tetrazol, Isothiazol, Oxazol, Isoxazol sowie davon abgeleitete ganz oder teilweise hydrierte Cyclen verstanden, wobei Oxazolin ausgenommen ist.

Die Aminosäuren A und B in Formel I sind durch eine Amidbindung miteinander verknüpft, es handelt sich um natürliche oder nichtnatürliche α-Aminosäuren der L-, D-oder D,L-Konfiguration, vorzugsweise der L-Konfiguration.

Unter Salzen von Verbindungen der Formel I sind insbesondere pharmazeutisch verwendbare oder nicht-toxische Salze zu verstehen.

Solche Salze werden beispielsweise von Verbindungen der Formel I, welche saure Gruppen, z.B. Carboxy, enthalten, mit Alkali- oder Erdalkalimetallen gebildet, wie Na, K, Mg und Ca, sowie mit physiologisch verträglichen organischen Aminen, wie z.B. Triethylamin und Tri-(2-hydroxy-ethyl)-amin.

Verbindungen der Formel I, welche basische Gruppen, z.B. eine Aminogruppe oder eine Guanidinogruppe, enthalten, bilden Salze mit anorganischen Säuren, wie z.B. Salzsäure, Schwefelsäure oder Phosphorsäure und mit organischen Carbon- oder Sulfonsäuren, wie z.B. Essigsäure, Citronensäure, Benzoesaure, Maleinsäure, Fumarsäure, Weinsäure und p-Toluolsulfonsäure.

$R^1$ bedeutet bevorzugt ($C_2$-$C_{11}$)-Alkanoyl, wie n-Decanoyl, Formyl, Acetyl, Pivaloyl, Isovaleryl oder Isobutyryl; substituiertes ($C_2$-$C_{11}$)Alkanoyl, wie 2-Hydroxypropionyl 2-Hydroxy-3-methyl-butyryl oder gegebenenfalls geschützes Amino-($C_2$-$C_{11}$)-alkanoyl, wie 4-Aminobutyryl, 5-Aminopentanoyl, 6-Aminohexanoyl, 4-N-tert.-Butoxycarbonylaminobutyryl, 5-N-tert.-Butoxycarbonylaminopentanoyl oder 6-N-tert.-Butoxycarbonylaminohexanoyl oder Di-($C_1$-$C_7$)-alkylamino-($C_2$-$C_{11}$)-alkanoyl, wie Dimethylaminoacetyl; ($C_4$-$C_9$)-Cycloalkykcarbonyl, wie Cyclopropylcarbonyl, Cyclobutylcarbonyl, Cyclopentylcarbonyl oder Cyclohexylcarbonyl; ($C_6$-$C_{10}$) Aryl-($C_2$-$C_{11}$)-alkanoyl, wie Phenylacetyl, Phenylpropanoyl oder Phenylbutanoyl, 2-(2,6-Dichloroanilino)-phenylacetyl, 2-(N-Benzyl-2,6-dichloranilino)-phenylacetyl; gegebenenfalls durch Halogen, ($C_1$-$C_7$)-Alkyl, ($C_1$-$C_7$)-Alkoxy oder ($C_1$-$C_7$)-Alkoxycarbonyl substituiertes Benzoyl, wie 4-Chlorbenzoyl, 4-Methylbenzoyl, 2 Methoxycarbonylbenzoyl oder 4-Methoxybenzoyl, Pyrrolyl-2-carbonyl, Pyridyl-3-carbonyl, Benzylsulfonyl; ($C_2$-$C_{11}$ -Alkoxycarbonyl, wie Methoxycarbonyl, Ethoxycarbonyl oder tert.-Butoxycarbonyl; durch Halogen substituiertes ($C_2$-$C_{11}$)-Alkoxycarbonyl, wie 2,2,2-Trichlorethoxycarbonyl oder 1,1-Dimethyl-2,2,2-trichlorethoxycarbonyl; ($C_6$-$C_{14}$)-Aryl-($C_2$-$C_7$)-alkoxycarbonyl, wie Benzyloxycarbonyl oder 9-Fluorenylmethylcarbonyl,

$R^2$ ist vorzugsweise Isobutyl, Benzyl oder Cyclohexylmethyl.

$R^3$ ist vorzugsweise Wasserstoff, Isopropyl oder Isobutyl.

Bevorzugte Aminosäuren A und B sind unabhängig voneinander Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, Lysin, Ornithin, 2,4-Diaminobuttersäure, Arginin, Norvalin, 4-Chlorphenylalanin, Methioninsulfon, Methioninsulfoxid, 2-Pyridylalanin, 3-Pyridylalanin, 4-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphtylalanin, 4-Nitrophenylalanin, Norleucin, Valin, Alanin, 1,2,3,4-Tetrahydroisochinolin-3-carbonsäure, Homophenylalanin, 2-Amino-4-(2-thienyl)buttersäure, (Z)-Dehydrophenylalanin oder (E)-Dehydrophenylalanin.

X ist bevorzugt abwesend.

n bedeutet bevorzugt 2 oder 3, bevorzugt 2.

$R^7$ steht bevorzugt für den Rest eines 5-gliedrigen Heterocyclus mit einem oder zwei Heteroatomen, der gegebenenfalls durch ($C_1$-$C_7$)-Alkyl substituiert ist.

Besonders bevorzugt steht $R^7$ für 2-Imidazolyl, 2-N-Methyl-imidazolyl, 2-N-Ethyl-imidazolyl, 2-N-n-Propyl-imidazolyl und 2-N-Isopropyl-imidazolyl.

Die Erfindung betrifft weiterhin ein Verfahren zur Herstellung von Verbindungen der Formel I, das dadurch gekennzeichnet ist, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

Fragmente einer Verbindung der Formel I mit einer endständigen Carboxylgruppe besitzen die nachstehenden Formeln IV a - IV c:

$R^1$-OH (IV a)

$R^1$-A-OH (IV b)

$R^1$-A-B-OH (IV c)

Fragmente einer Verbindung der Formel I mit einer endständigen Aminogruppe besitzen die nachstehenden Formeln V a - V c:

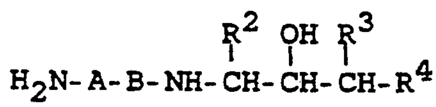

(V a)

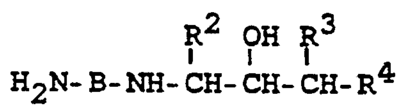

(V b)

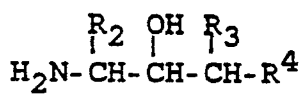

(V c)

Methoden, die zur Herstellung einer Amidbindung geeignet sind, werden z.B. in Houben-Weyl, Methoden der organischen Chemie, Band 15/2; Bodanszky et al., Peptide synthesis, 2nd ed. (Wiley & Sons, New York 1976) oder Gross, Meienhofer, The Peptides. Analysis, synthesis, biology (Academic Press, New York 1979) beschrieben. Vorzugsweise werden die folgenden Methoden herangezogen: Aktivestermethode mit N-Hydroxy-succinimid als Esterkomponente, Kupplung mit einem Carbodiimid wie Dicyclohexylcarbodiimid oder mit Propanphoshonsäureanhydrid und die Gemischt-Anhydrid-Methode mit Pivaloylchlorid.

Die Herstellung der als Ausgangsverbindung verwendeten optisch aktiven Amine der Formel V c

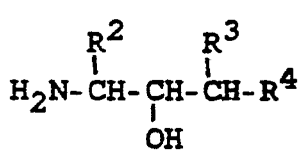

(V c)

worin $R^2$, $R^3$ und $R^4$ wie oben definiert sind, erfolgt ausgehend von optisch aktiven α-Aminosäuren, wobei deren Asymmetriezentrum erhalten bleibt. Hierzu wird in bekannter Weise ein N-geschützter Aminosäurealdehyd hergestellt, welcher nach einer Aldol-Addition in einen entsprechenden Heteroarylalkyl-Baustein umgewandelt wird. Nach Abspaltung der N-Schutzgruppe werden Aminoalkohole der Formel V c erhalten. Man erhält Diastereomerengemische bezüglich des OH-tragenden Zentrums, die in an sich bekannter Weise, beispielsweise durch fraktionierte Kristallisation oder durch Chromatographie getrennt werden. Die

Überprüfung der Diastereomerenreinheit erfolgt, mittels HPLC, die Enantiomerenreinheit kann in bekannter Weise durch Überführung in Mosher-Derivate durchgeführt werden (H.S. Mosher et. al., J. org. Chem. 34, 2543 (1969)).

Die Herstellung N-geschützter Aminosäurealdehyde erfolgt nach B. Castro et al. (Synthesis 1983, 676).

Die Aldol-analoge Addition an N-geschützte Aminosäurealdehyde (bevorzugterweise N-tert.-Butoxycarbonyl-und Benzyloxycarbonyl Schutzgruppen) erfolgt in einem gegenüber Basen inerten Lösungsmittel, wie Ether, THF, Toluol, DMF, DMSO oder Dimethoxyethan.

Als Basen zur Deprotonierung der Heteroarylalkyl-Komponente können Alkalimetallalkoholate, wie Kalium-O-tert.-butylat, Natriummethylat, Alkalimetallhydride, wie Natrium- oder Kaliumhydrid, metallorganische Basen, wie n-Butyllithium, s-Butyllithium, Methyllithium oder Phenyllithium, Natriumamid sowie Alkalimetallsalze von organischen Stickstoffbasen, wie Lithiumdiisopropylamid verwendet werden.

Die substituierten 4-Amino-3-hydroxybuttersäuren sind literaturbekannt und werden nach D.H. Rich et. al., J. Org. Chem. 43, 3624 (1978) hergestellt.

Die zur Herstellung von Verbindungen der Formel I erforderlichen Vor- und Nachoperationen wie Einführung und Abspaltung von Schutzgruppen sind literaturbekannt und sind z.B. in T.W. Greene, "Protective Groups in Organic Synthesis" beschrieben. Salze von Verbindungen der Formel I mit salzbildenden Gruppen werden in an sich bekannter Weise hergestellt, indem man z.B. eine Verbindung der Formel I mit einer basischen Gruppe mit einer stöchiometrischen Menge einer geeigneten Säure umsetzt. Stereoisomerengemische insbesondere Diastereomerengemische, die bei Verwendung racemischer Aminosäuren A oder B anfallen, können in an sich bekannter weise durch fraktionierte Kristallisation oder durch Chromatographie getrennt werden.

Die erfindungsgemäßen Verbindungen der Formel I weisen enzymhemmende Eigenschaften auf; insbesondere hemmen sie die Wirkung des natürlichen Enzyms Renin. Renin ist ein proteolytisches Enzym aus der Klasse der Aspartyl-Proteasen welches als Folge verschiedener Stimuli (Volumendepletion, Natriummangel, $\beta$-Rezeptorenstimulation) von den juxtaglomerulären Zellen der Niere in den Blutkreislauf sezerniert wird. Dort spaltet es von dem aus der Leber ausgeschiedenen Angiotensinogen das Decapeptid Angiotensin 1 ab. Dieses wird durch das "angiotensin converting enzyme" (ACE) in Angiotensin 2 überführt. Angiotension 2 spielt eine wesentliche Rolle bei der Blutregulation, da es direkt den Blutdruck durch Gefäßkontraktion steigert. Zusätzlich stimuliert es die Sekretion von Aldosteron aus der Nebenniere und erhöht auf diese Weise über die Hemmung der Natrium-Ausscheidung das extrazelluläre Flüssigkeitsvolumen, was seinerseits zu einer Blutdrucksteigerung beiträgt. Hemmen der enzymatischen Aktivität des Renins bewirken eine verminderte Bildung von Angiotensin 1, was eine verminderte Bildung von Angiotensin 2 zur Folge hat. Die Erniedrigung der Konzentration dieses aktiven Peptidhormons ist die direkte Ursache für die blutdrucksenkende Wirkung von Renin-Hemmern.

Die Wirksamkeit von Renin-Hemmern kann durch in-vitro-Tests überprüft werden. Hierbei wird die Verminderung der Bildung von Angiotensin 1 in verschiedenen Systemen (Humanplasma, Schweine-Renin) gemessen. Hierzu wird z.B. Humanplasma, welches sowohl Renin als auch Angiotensinogen enthält, bei 37° C mit der zu testenden Verbindung inkubiert. Anschließend wird die Konzentration des während der Inkubation gebildeten Angiotensin 1 mit einem Radioimmunoassay gemessen. Die in der vorliegenden Erfindung beschriebenen Verbindungen der allgemeinen Formel I zeigen in verwendeten in-vitro-Tests Hemmwirkungen bei Konzentrationen von etwa $10^{-5}$ bis $10^{-10}$ Mol/l.

Renin-Hemmer bewirken an salzverarmten Tieren eine Blutdrucksenkung. Da sich menschliches Renin von dem Renin anderer Spezies unterscheidet, werden zum in-vivo-Test von Renin-Hemmern Primaten (Marmosets, Rhesus-Affen) herangezogen. Primaten-Renin und Human-Renin sind in ihrer Sequenz weitgehend homolog. Durch i.v. Injektion von Furosemid wird eine endogene Renin-Ausschüttung angeregt. Anschließend werden die Testverbindungen durch kontinuierliche Infusion verabreicht und ihre Wirkung auf Blutdruck und Herzfrequenz wird gemessen. Die Verbindungen der vorliegenden Erfindung sind hierbei in einem Dosisbereich von etwa 0,1 - 5 mg/kg i.v. wirksam. Die in der vorliegenden Erfindung beschriebenen Verbindung der allgemeinen Formel I können als Antihypertensiva, sowie zur Behandlung der Herzinsuffizienz verwendet werden.

Die Erfindung betrifft daher auch die Verwendung von Verbindungen der Formel I als Heilmittel und pharmazeutische Präparate, die diese Verbindung enthalten. Bevorzugt ist die Anwendung bei Primaten, insbesondere beim Menschen.

Pharmazeutische Präparate enthalten eine wirksame Menge des Wirkstoffs der Formel I zusammen mit einem anorganischem oder organischen pharmazeutisch verwendbaren Trägerstoff. Die Anwendung kann intranasal intravenös, subkutan oder peroral erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-,

Misch-, Granuliert- oder Dragierverfahren hergestellt.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zübereitung sowohl als Trocken- oder Feuchtgranulat erfolgen. Als ölige Trägerstoffe oder Lösungsmittel kommen beispielsweise pflanzliche oder tierische Öle in Betracht, wie Sonnenblumenöl und Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösung, Suspensionen oder Emulsionen gebracht. Als Lösungsmittel kommen z.B. in Frage: Wasser, physiologische Kochsalzlösungen oder Alkohole, z.B. Ethanol, Propandiol oder Glycerin, daneben auch Zuckerlösungen wie Glucose- oder Mannitlösungen, oder auch eine Mischung aus den verschiedenen genannten Lösungsmitteln.

| Verzeichnis der verwendeten Abkürzungen : | |
|---|---|
| Ac | Acetyl |
| ACHPA | [3S,4S]-4-Amino-3-hydroxy-5-cyclohexylpentansäure |
| BOC | tert.-Butoxycarbonyl |
| DC | Dünnschichtchromatographie |
| DCC | Dicyclohexylcarbodiimid |
| DNP | 2,4-Dinitrophenyl |
| DMF | Dimethylformamid |
| DMSO | Dimethylsulfoxid |
| EE | Essigsaüreethylester |
| Etoc | Ethoxycarbonyl |
| FAB | Fast atom bombardment |
| H | Hexan |
| HOBt | 1-Hydroxybenzotriazol |
| Iva | Isovaleryl |
| M | Molekularpeak |
| MeOH | Methanol |
| MS | Massenspektrum |
| MTB | Methyl-tert.-butylether |
| R.T. | Raumtemperatur |
| Schmp. | Schmelzpunkt |
| Thi | β-2-Thienylalanin |
| THF | Tetrahydrofuran |
| Z | Benzyloxycarbonyl |

Die sonstigen für Aminosäuren verwendeten Abkürzungen entsprechen dem in der Peptidchemie üblichem drei Buchstaben-Code wie er z.B. in Europ. J. Biochem. 138, 9 - 37 (1984) beschrieben ist. Falls nicht ausdrücklich anders angegeben, handelt es sich immer um Aminosäuren der L-Konfiguration.

Die nachstehenden Beispiele dienen zur Erläuterung der vorliegenden Erfindung, ohne daß diese darauf beschränkt wäre.

**Beispiel 1**

Iva-Phe-His-1(S)-cyclohexylmethyl-2(S)-hydroxy-5-(2-imidazolyl)-pentylamid

70 mg Iva-Phe-His-1(S)-cyclohexylmethyl-2(S)-hydroxy-5-(2-N-benzyloxymethyl-imidazolyl)-pentylamid werden in 15 ml Ethanol gelöst. Nach Zugabe von 35 mg 10 %igem Palladium auf Aktivkohle und 1,5 ml Eisessig wird 20 h bei Raumtemperatur und Normaldruck hydriert. Nach Filtration wird eingeengt. Nach

Zugabe von 20 ml gesättigter $NaHCO_3$- Lösung wird 3 mal mit EE extrahiert. Nach Trocknen der EE-Phasen mit $Na_2SO_4$ wird eingeengt, wobei die Titelverbindung als schwach gelbe Substanz anfällt.
$R_f$ (EE/MeOH 2/1) = 0,1
MS(FAB) = 634 (M+1).

a) Iva-Phe-His-1(S)-cyclohexylmethyl-2(S)-hydroxy-5-(2-N-benzyloxymethyl-imidazolyl)-pentylamid.

200 mg Iva-Phe-His(DNP)-1(S)-cyclohexylmethyl-2(S)-hydroxyl-5-(2 N-benzyloxymethyl-imidazolyl)-pentylamid werden in 5 ml Acetonitril mit 0,5 ml Thiophenol 3 h bei Raumtemperatur gerührt. Man engt im Vakuum ein und digeriert 3 mal mit Diisopropylether. Nach Chromatographie an Kieselgel (Laufmittel EE/Methanol 10:1) und Einengen der produkhaltigen Fraktionen erhält man ein farbloses Pulver.
$R_f$ (EE/MeOH 10/1) = 0,2
MS(FAB) = 754 (M+1).

b) Iva-Phe-His(DNP)-1(S)-cyclohexylmethyl-2(S)-hydroxy-5-(2-N-benzyloxymethylimidazolyl)-pentylamid.

Zu 192 mg Iva-Phe-His(DNP)-OH, 30 µl Pyridin und 50 µl N-Ethylpiperidin in 10 ml $CH_2Cl_2$ wird bei -5 °C 44 µl Pivaloylchlorid zugegeben. 30 min wird bei + 5 bis + 10 °C gerührt, auf - 10 °C gekühlt und 1-(S)-cyclohexylmethyl-2(S)-hydroxy-5-(2-N-benzyloxymethyl-imidazolyl)-pentylamin in 5 ml $CH_2Cl_2$ zugetropft. Man rührt 16 h ohne Kühlung, engt im Vakuum ein und nimmt in 100 ml EE auf. Man extrahiert 3 mal mit wäßriger $K_2CO_3$-Lösung, 1 mal mit $H_2O$, trocknet über $Na_2SO_4$ und engt im Vakuum ein. Nach Chromathographie an Kieselgel (Laufmittel EE/Methanol 10:1) erhält man die Titelverbindung als schwach gelbliches Pulver.
$R_f$ (EE/Methanol 10/1) = 0,4
MS(FAB) = 920 (M+1)

c) 1(S)-Cyclohexylmethyl-2(S)-hydroxy-5-[2-N-benzyloxymethyl-imidazolyl]-pentylamin

500 mg 3-Boc-4(S)-cyclohexylmethyl-2,2-dimethyl-5-[3-(2-N-benzyloxymethyl-imidazolyl)-propyl)]-oxazolidin werden in 15 ml Dioxan gelöst. Unter leichter Kühlung (+ 10 °C) werden 10 ml 12 %ige HCl in Dioxan zugegeben. Nach 3 h bei Raumtemperatur wird im Vakuum eingeengt, in $H_2O$ aufgenommen, mit gesättigter wäßriger $Na_2CO_3$-Lösung auf pH = 9-10 eingestellt und 3 mal mit EE extrahiert. Nach Trocknung mit $Na_2SO_4$ und Einengen im Vakuum erhält man die Titelverbindung, die ohne weitere Reinigung in den nächsten Kupplungsschritten verwendet werden kann.

d) 3-Boc-4(S)-cyclohexylmethyl-2,2-dimethyl-5-[3-(2-N-benzyloxymethyl-imidazolyi)-propyl)]-oxazolidin

550 mg 2-Methyl-N-benzyloxymethyl-imidazol werden in 5 ml trockenem THF gelöst. Unter Argon-Atmosphäre gibt man bei -60° 1,9 ml 1,5 M n-Butyllithium in Hexan zu. Nach 15 min addiert man 500 mg 3-Boc-4(S)-cyclohexylmethyl-2,2-dimethyl-5-(2-bromethyl)-oxazolidin in 5 ml THF. Nach 1 h bei -60 °C gibt man 10 ml gesättigte $NaHCO_3$-Lösung zu. Nach Aufwärmen auf Raumtemperatur wird 3 mal mit EE extrahiert, mit $Na_2SO_4$ getrocknet und eingeengt. Die Titelverbindung erhält man durch Chromatographie an Kieselgel (Laufmittel EE).
$R_f$ (EE) = 0,3
MS(FAB) = 526 (M+1)

e) 2-Methyl-N-benzyloxymethyl-imidazol

5 g 2-Methylimidazol werden portionsweise zu 16,8 g Kaliumhydroxid in 200 ml trockenem Aceton zugegeben. Nach 5 min tropft man bei Raumtemperatur 8,5 ml Benzyloxymethylchlorid zu. Nach 15 min wird eingeengt und mit 2N HCl auf pH = 1 umgestellt. Nun wird 3 mal mit EE extrahiert, die organischen Phasen verworfen. Die wässrige Phase wird nun mit KOH auf pH ~ 9-10 gestellt und 3 x mit EE extrahiert. Die vereinten EE-Phasen werden mit $Na_2SO_4$ getrocknet und eingeengt. Chromatographie an Kieselgel

(Eluens EE/MeOH 10/1) liefert die Titelverbindung.
$R_f$ (EE/MeOH 10/1) = 0,4

f) 3-Boc-4(S)-cyclohexylmethyl-2,2-dimethyl-5-(2-bromethyl)-oxazolidin

Zu 690 mg 3-Boc-4(S)-cyclohexylmethyl-2,2-dimethyl-5-(2-hydroxyethyi)-oxazolidin, 2,6 g Triphenylphosphin und 1,6 g Pyridiniumhydrobromid in 15 ml $CH_2Cl_2$ werden unter Argon 1,6 ml Azodicarbonsäurediethylester bei 20 °C zugetropft. Nach 16 h bei R.T. wird $H_2O$ zugegeben und mit 100 ml $CH_2Cl_2$ verdünnt. Die organische Phase wird 2 mal mit gesättigter $NaHCO_3$-Lösung und 1 mal mit gesättigter NaCl-Lösung gewaschen. Die mit $Na_2SO_4$ getrocknete organische Phase wird eingeengt, der Rückstand in wenig EE aufgenommen und zur Abtrennung von PPh3 filtriert. Reinigung an Kieselgel liefert die Titelverbindung (Eluens: H/EE 15:1).
$R_f$ (H/EE 15/1) = 0,3; MS 404 (M)

g) 3-Boc-4(S)-cyclohexylmethyl-2,2-dimethyl-5-(2-hydroxyethyl)-oxazolidin

10 g Boc-ACHPA-OEt, (Herstellung nach J.Med. Chem. 28 [1985], 1779) 500 mg p-Toluolsulfonsäure und 7,2 ml Dimethoxypropan werden in 160 ml Toluol unter Argon 2 h auf 80 °C erhitzt. Anschließend wird eingeengt. Der Rückstand wird bei 0 °C zu einer Suspension aus 2 g $LiAlH_4$ in 200 ml THF zugetropft. Nach 2,5 h bei 0 °C werden 100 ml 5 %ige $NaHSO_4$-Lösung zugegeben und 3 mal mit EE extrahiert. Die vereinigten organischen Phasen werden 1 mal mit gesättigter $NaHCO_3$-Lösung gewaschen. Nach dem Trocknen mit $Na_2SO_4$ wird eingeengt und chromatographiert (Eluens: H/EE 2:1).
$R_f$ (H/EE 4:1) = 0,1; MS = 342 (M+1)

Beispiele 2- 10 werden analog Beispiel 1 hergestellt

**Beispiel 2**
Iva-Phe-His-1(S)-cyclohexylmethyl-2(S)-hydroxy-5-(2-N-methyl-imidazolyl)-pentylamid
$R_f$ (EE(MeOH 3/1) = 0.1
MS(FAB) = 64B (M+1)

**Beispiel 3**
Iva-Phe-His-1(S)-cyclohexylmethyl-2(S)-hydroxy-5-(2-N-ethyl-imidazolyl)-pentylamid
$R_f$ ($CH_2Cl_2$/$NH_4OH$/MeOH 10/0,1/1) = 0,35
MS(FAB) = 662 (M+1)

**Beispiel 4**
Iva-Phe-His-1(S)-cyclohexylmethyl-2(S)-hydroxy-5-(2-N-n-propyl-imidazolyl)-pentylamid
$R_f$ (EE/MeOH 3/1) = 0,1
MS(FAB) = 676 (M+1)

**Beispiel 5**
Iva-Phe-His-1(S)-cyclohexylmethyl-2(S)-hydroxy-5-(2-N-isopropyl-imidazolyl)-pentylamid
$R_f$ (EE/MeOH 3/1) = 0,1
MS(FAB) = 676 (M+1)

Die Darstellung der 2-Methyl-N-alkyl-imidazole ist im Folgenden am Beispiel des 2-Methyl-N-n-propyl-imidazols beschrieben:

2-methyl-N-n-propyl-imidazol
10 g 2-Methylimidazol werden in 40 ml n-Propylbromid 2 h zum Sieden erhitzt. Anschließend wird auf 150 ml 5 %ige $NaHSO_4$-Lösung gegossen, 3 mal mit EE extrahiert, mit $Na_2SO_4$ getrocknet und eingeengt. Der Rückstand wird in Ether aufgenommen und von unlöslichem Ausgangsmaterial durch Filtration abgetrennt. Auf diese Weise fällt die Titelverbindung als farbloses Öl an.
$R_f$ (EE/MeOH 10/1) = 0,2

**Beispiel 6**
Iva-Phe-Nva-1(S)-cyclohexylmethyl-2(S)-hydroxy-5-(2-imidazolyl)-pentylamid
$R_f$ (EE/MeOH 5/1) = 0,1
MS(FAB) = 596 (M+1)

**Beispiel 7**
Iva-Phe-Nva-1(S)-cyclohexylmethyl-2(S)-hydroxy-5-(2-N-methyl-imidazolyl)-pentylamid
$R_f$ ($CH_2Cl_2$/MeOH/$NH_4OH$ 20/1/0,1) = 0,15
MS(FAB) = 610 (M+1)

**Beispiel 8**
Iva-Phe-Nva-1(S)-cyclohexylmethyl-2(S)-hydroxy-5-(2-N-ethyl-imidazolyl)-pentylamid
$R_f$ ($CH_2Cl_2$/MeOH/$NH_4OH$ 20/1/0,1) = 0,15
MS(FAB) = 624 (M+1)
**Beispiel 9**
Iva-Phe-Nva-1(S)-cyclohexylmethyl-2(S)-hydroxy-5-(2-N-n-propyl-imidazolyl)-pentylamid
$R_f$ (EE/MeOH 10/1) = 0,3
MS(FAB) = 638 (M+1)
**Beispiel 10**
Ival-Phe-Nva-1(S)-cyclohexylmethyl-2(S)-hydroxy 5(2-N-isopropyl-imidazolyl)-pentylamid
$R_f$ (EE/MeOH 10/1) = 0,3
MS(FAB) = 638 (M+1)

## Ansprüche

1. Verbindung der Formel I

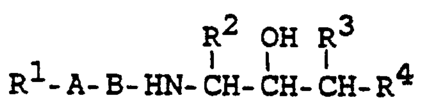

(I)

in welcher
$R^1$ einen Rest der Formel II bedeutet,
$R^a$-W- (II)
worin W für -CO-, -O-CO-, $-SO_2-$ oder -NH-CO- steht und
$R^a$ Wasserstoff, ($C_1$-$C_{10}$)-Alkyl, das gegebenenfalls ein-oder zweifach ungesättigt ist und das gegebenenfalls durch bis zu 3 gleiche oder verschiedene Reste aus der Reihe Hydroxy, ($C_1$-$C_7$)-Alkoxy, ($C_1$-$C_7$)-Alkanoyloxy, Carboxy, ($C_1$-$C_7$)-Alkoxycarbonyl, Halogen, Amino, ($C_1$-$C_7$)-Alkylamino, Di-($C_1$-$C_7$)-alkylamino, ($C_1$-$C_5$)-Alkoxycarbonylamino, ($C_7$-$C_{15}$)-Aralkoxycarbonylamino und 9-Fluorenylmethyloxycarbonylamino substituiert ist, ($C_3$-$C_8$)-Cycloalkyl, ($C_3$-$C_8$)-Cycloalkyl-($C_1$-$C_6$)-alkyl, ($C_6$-$C_{14}$)-Aryl, das gegebenfalls durch einen oder zwei gleiche oder verschiedene Reste aus der Reihe F, Cl, Br, J, Hydroxy, ($C_1$-$C_7$)-Alkoxy, ($C_1$-$C_7$)-Alkyl, ($C_1$-$C_7$)-Alkoxycarbonyl, Amino, gegebenenfalls mit bis zu 2 Halogen substituiertes Anilino und Trifluormethyl substituiert ist,
($C_6$-$C_{14}$)-Aryl-($C_1$-$C_6$)-alkyl, worin der Arylteil gegebenenenfalls durch einen oder zwei gleiche oder verschiedene Reste aus der Reihe F, Cl, Br, J, Hydroxy, ($C_1$-$C_7$)-Alkoxy, ($C_1$-$C_7$)-Alkyl, ($C_1$-$C_7$)-Alkoxycarbonyl, Amino, ($C_1$-$C_7$)-Alkylamino, Di-($C_1$-$C_7$)-alkylamino, Carboxy, Carboxymethoxy, Amino-($C_1$-$C_7$)-alkyl, ($C_1$-$C_7$)-Alkylamino-($C_1$-$C_7$)-alkyl, Di-($C_1$-$C_7$)-alkylamino-($C_1$-$C_7$)-alkyl, ($C_1$-$C_7$)-Alkoxycarbonylmethoxy, Carbamoyl, Sulfamoyl, ($C_1$-$C_7$)-Alkoxysulfonyl, Sulfo und Guanidinomethyl substituiert ist, oder für den Rest eines 5- oder 6-gliedrigen monocyclischen oder 9- oder 10-gliedrigen bicyclischen Heterocyclus mit mindestens 1 C-Atom, 1 -4 N-Atomen und/oder 1 S- oder O-Atom auch als Ringglieder steht, der gegebenenfalls durch einen oder mehrere Reste aus der Reihe F, Cl, Br, Hydroxy, ($C_1$-$C_7$)-Alkoxy, ($C_1$-$C_7$)-Alkyl, ($C_2$-$C_8$)-Alkoxycarbonyl, Amino oder Trifluormethyl, di- oder trisubstituiert ist,
$R^4$ einen Rest der Formel III bedeutet,
$-X-(CH_2)_n-R^7$ (III)
A einen N-terminal mit $R^1$ und C-terminal mit B verknüpften Rest einer Aminosäure aus der Reihe Phenylalanin, Histidin, Tyrosin, Tryptophan, Methionin, Leucin, Isoleucin, Asparagin, Asparaginsäure, $\beta$-2-Thienylalanin, $\beta$-3-Thienylalanin, $\beta$-2-Furanylalanin, $\beta$-3-Furanylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, 4-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, $\beta$-2-Benzo(b)thienylalanin, $\beta$-3-Benzo(b)thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyldopa, 2-Amino-4-(2-thienyl)-buttersäure, 2-Amino-4-(3-thienyl)-buttersäure, 3-(2-Thienyl)-serin, (Z)-Dehydrophenylalanin und (E)-Dehydrophenylalanin bedeutet,
B einen Rest einer Aminosäure, wie unter A definiert, bedeutet,

$R^2$ Wasserstoff, ($C_1$-$C_{10}$)-Alkyl, ($C_4$-$C_7$)-Cycloalkyl, ($C_4$-$C_7$)-Cycloalkyl-($C_1$-$C_4$)-alkyl, ($C_6$-$C_{14}$)-Aryl oder ($C_6$-$C_{14}$)-Aryl-($C_1$-$C_4$)-alkyl bedeutet

$R^3$ Wasserstoff ($C_1$-$C_{10}$)-Alkyl, ($C_6$-$C_{14}$)-Aryl oder ($C_6$-$C_{14}$)-Aryl-($C_1$-$C_4$)-alkyl bedeutet,

X wahlweise abwesend sein kann oder O oder S bedeutet,

n = 2, 3 oder 4 sein kann und

$R^7$ Hydroxy oder Amino bedeutet oder für den Rest eines 5-gliedrigenHeterocyclus mit mindestens 1 C-Atom, 1-4 N-Atomen und/oder 1 S- oder O-Atom, der gegebenenfalls durch einen oder mehrere Reste aus der Reihe F, Cl, Br, Hydroxy, ($C_1$-$C_7$)-Alkoxy, ($C_1$-$C_7$)-Alkyl, ($C_2$-$C_8$)-Alkoxycarbonyl, Amino oder Trifluormethyl substituiert ist, steht, wobei Oxazolin ausgenommen ist.

sowie deren physiologisch verträglichen Salze.

2. Verbindung gemäß Anspruch 1, dadurch gekennzeichnet, daß

$R^1$ ($C_2$-$C_{11}$)-Alkanoyl, wie n Decanoyl, Formyl, Acetyl, Pivaloyl, Isovaleryl oder Isobutyryl; substituiertes ($C_2$-$C_{11}$)Alkanoyl, wie 2-Hydroxypropionyl 2-Hydroxy 3-methyl-butyryl oder gegebenenfalls geschütezs Amino-($C_2$-$C_{11}$)-alkanoyl, wie 4-Aminobutyryl, 5-Aminopentanoyl, 6-Aminohexanoyl, 4-N-tert.-Butoxycarbonylaminobutyryl, 5-N-tert.-Butoxycarbonylaminopentanoyl oder 6-N-tert.-Butoxycarbonylaminohexanoyl oder Di-($C_1$-$C_7$)-alkylamino-($C_2$-$C_{11}$)-alkanoyl, wie Dimethylaminoacetyl; ($C_4$-$C_9$)-Cycloalkylcarbonyl, wie Cyclopropylcarbonyl, Cyclobutylcarbonyl, Cyclopentylcarbonyl oder Cyclohexylcarbonyl; ($C_6$-$C_{10}$)-Aryl-($C_2$-$C_{11}$)-alkanoyl, wie Phenylacetyl, Phenylpropanoyl oder Phenylbutanoyl, 2-(2,6-Dichloroanilino)-phenylacetyl, 2-(N-Benzyl-2,6-dichloranilino)-phenylacetyl; gegebenenfalls durch Halogen, ($C_1$-$C_7$)-Alkyl, ($C_1$-$C_7$)-Alkoxy oder ($C_1$-$C_7$)-Alkoxycarbonyl substituiertes Benzoyl, wie 4-Chlorbenzoyl, 4-Methylbenzoyl, 2-Methoxycarbonylbenzoyl oder 4-Methoxybenzoyl, Pyrrolyl-2-carbonyl, Pyridyl-3-carbonyl, Benzylsulfonyl; ($C_2$-$C_{11}$ -Alkoxycarbonyl, wie Methoxycarbonyl, Ethoxycarbonyl oder tert.-Butoxycarbonyl; durch Halogen substituiertes ($C_2$-$C_{11}$)-Alkoxycarbonyl, wie 2,2,2-Trichlorethoxycarbonyl oder 1,1-Dimethyl-2,2,2-trichlorethoxycarbonyl; ($C_6$-$C_{14}$)-Aryl-($C_2$-$C_7$)-alkoxycarbonyl, wie Benzyloxycarbonyl oder 9-Fluorenylmethylcarbonyl bedeutet.

3. Verbindung gemäß Anspruch 1 und/oder 2, dadurch gekennzeichnet, daß $R^2$ Isobutyl, Benzyl oder Cyclohexylmethyl bedeutet.

4. Verbindung gemäß einem oder mehreren der Ansprüche 1 bis 3, dadurch gekennzeichnet, daß $R^3$ Wasserstoff, Isopropyl oder Isobutyl bedeutet.

5. Verbindung gemäß einem oder mehreren der Ansprüche 1 bis 4, dadurch gekennzeichnet, daß A und B unabhängig voneinander Phenylalanin, Histidin, Tyrosin, Tryptophan, Methionin, Leucin, Isoleucin, Asparagin, Asparaginsäure, $\beta$-2-Ihienylalanin, $\beta$-3-Thienylalanin, $\beta$-2-Furylalanin, Lysin, Ornithin, 2,4-Diaminobuttersäure, Arginin, Norvalin, 4-Chlorphenylalanin, Methioninsulfon, Methioninsulfoxid, 2-Pyridylalanin, 3-Pyridylalanin, 4-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphtylalanin, 4-Nitrophenyialanin, Norleucin, Valin, Alanin, 1,2,3,4-Tetrahydroisochinolin-3-carbonsäure, Homophenylalanin, 2-Amino-4-(2-thienyl)-buttersäure, (Z)-Dehydrophenylalanin oder (E)-Dehydrophenylalanin bedeuten.

6. Verbindung gemäß einem oder mehreren der Ansprüche 1 bis 5, dadurch gekennzeichnet, daß $R^7$ für den Rest eines 5-gliedrigen Heterocyclus mit einem oder zwei Heteroatomen, der gegebenenfalls durch ($C_1$-$C_7$) Alkyl substituiert ist, steht.

7. Verfahren zur Herstellung einer Verbindung der Formel I gemäß einem der Ansprüche 1 bis 6, dadurch gekennzeichnet, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

8. Verwendung einer Verbindung gemäß einem der Ansprüche 1 bis 6 als Heilmittel.

9. Verwendung einer Verbindung gemäß einem der Ansprüche 1 bis 6 als Heilmittel bei der Behandlung des Bluthochdrucks und der Herzinsuffizienz.

10. Pharmazeutisches Mittel enthaltend eine Verbindung gemäß einem der Ansprüche 1 bis 6.

Patentansprüche für folgende Vertragsstaaten: ES, GR

1. Verfahren zur Herstellung einer Verbindung der Formel I

$$\begin{array}{c} \qquad\qquad\qquad R^2 \quad OH \quad R^3 \\ R^1\text{-}A\text{-}B\text{-}HN\text{-}CH\text{-}CH\text{-}CH\text{-}R^4 \end{array} \qquad (I)$$

in welcher
$R^1$ einen Rest der Formel II bedeutet,
$R^a$-W- (II)
worin W für -CO-, -O-CO-, $-SO_2-$ oder -NH-CO- steht und
$R^a$ Wasserstoff, ($C_1$-$C_{10}$)-Alkyl, das gegebenenfalls ein-oder zweifach ungesättigt ist und das gegebenenfalls durch bis zu 3 gleiche oder verschiedene Reste aus der Reihe Hydroxy, ($C_1$-$C_7$)-Alkoxy, ($C_1$-$C_7$)-Alkanoyloxy, Carboxy, ($C_1$-$C_7$)-Alkoxycarbonyl, Halogen, Amino, ($C_1$-$C_7$)-Alkylamino, Di-($C_1$-$C_7$)-alkylamino, ($C_1$-$C_5$)-Alkoxycarbonylamino, ($C_7$-$C_{15}$)-Aralkoxycarbonylamino und 9-Fluorenylmethyloxycarbonylamino substituiert ist, ($C_3$-$C_8$)-Cycloalkyl, ($C_3$-$C_8$)-Cycloalkyl-($C_1$-$C_6$)-alkyl, ($C_6$-$C_{14}$)-Aryl, das gegebenfalls durch einen oder zwei gleiche oder verschiedene Reste aus der Reihe F, Cl, Br, J, Hydroxy, ($C_1$-$C_7$)-Alkoxy, ($C_1$-$C_7$)-Alkyl, ($C_1$-$C_7$)-Alkoxycarbonyl, Amino, gegebenenfalls mit bis zu 2 Halogen substituiertes Anilino und Trifluormethyl substituiert ist,
($C_6$-$C_{14}$)-Aryl-($C_1$-$C_6$)-alkyl, worin der Arylteil gegebenenenfalls durch einen oder zwei gleiche oder verschiedene Reste aus der Reihe F, Cl, Br, J, Hydroxy, ($C_1$-$C_7$)-Alkoxy, ($C_1$-$C_7$)-Alkyl, ($C_1$-$C_7$) Alkoxycarbonyl, Amino, ($C_1$-$C_7$)-Alkylamino, Di-($C_1$-$C_7$)-alkylamino, Carboxy, Carboxymethoxy, Amino-($C_1$-$C_7$)-alkyl, ($C_1$-$C_7$)-Alkylamino-($C_1$-$C_7$)-alkyl, Di-($C_1$-$C_7$)-alkylamino-($C_1$-$C_7$)-alkyl, ($C_1$-$C_7$)-Alkoxycarbonylmethoxy, Carbamoyl, Sulfamoyl, ($C_1$-$C_7$)-Alkoxysulfonyl, Sulfo und Guanidinomethyl substituiert ist, oder für den Rest eines 5- oder 6-gliedrigen monocyclischen oder 9- oder 10-gliedrigen bicyclischen Heterocyclus mit mindestens 1 C-Atom, 1 -4 N-Atomen und/oder 1 S- oder O-Atom auch als Ringglieder steht, der gegebenenfalls durch einen oder mehrere Reste aus der Reihe F, Cl, Br, Hydroxy, ($C_1$-$C_7$)-Alkoxy, ($C_1$-$C_7$)-Alkyl, ($C_2$-$C_8$)-Alkoxycarbonyl, Amino oder Trifluormethyl, di- oder trisubstituiert ist,
$R^4$ einen Rest der Formel III bedeutet,
$- X -(CH_2)_n -R^7$ (III)
A einen N-terminal mit $R^1$ und C-terminal mit B verknüpften Rest einer Aminosäure aus der Reihe Phenylalanin, Histidin, Tyrosin, Tryptophan, Methionin, Leucin, Isoleucin, Asparagin, Asparaginsäure, $\beta$-2-Thienylalanin, $\beta$-3-Thienylalanin, $\beta$-2-Furanylalanin, $\beta$-3-Furanylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, 4-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, $\beta$-2-Benzo(b)thienylalanin, $\beta$-3-Benzo(b)thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyldopa, 2-Amino-4-(2-thienyl)-buttersäure, 2-Amino-4-(3-thienyl)-buttersäure, 3-(2-Thienyl)-serin, (Z)-Dehydrophenylalanin und (E)-Dehydrophenylalanin bedeutet,
B einen Rest einer Aminosäure, wie unter A definiert, bedeutet,
$R^2$ Wasserstoff, ($C_1$-$C_{10}$)-Alkyl, ($C_4$-$C_7$)-Cycloalkyl, ($C_4$-$C_7$)-Cycloalkyl-($C_1$-$C_4$)-alkyl, ($C_6$-$C_{14}$)-Aryl oder ($C_6$-$C_{14}$)-Aryl-($C_1$-$C_4$)-alkyl bedeutet
$R^3$ Wasserstoff ($C_1$-$C_{10}$)-Alkyl ($C_6$-$C_{14}$)-Aryl oder ($C_6$-$C_{14}$)-Aryl-($C_1$-$C_4$)-alkyl bedeutet,
X wahlweise abwesend sein kann oder O oder S bedeutet,
n = 2, 3 oder 4 sein kann und
$R^7$ Hydroxy oder Amino bedeutet oder für den Rest eines 5-gliedrigenHeterocyclus mit mindestens 1 C-Atom, 1-4 N-Atomen und/oder 1 S- oder O-Atom, der gegebenenfalls durch einen oder mehrere Reste aus der Reihe F, Cl, Br, Hydroxy, ($C_1$-$C_7$)-Alkoxy, ($C_1$-$C_7$)-Alkyl, ($C_2$-$C_8$)-Alkoxycarbonyl, Amino oder Trifluormethyl substituiert ist, steht, wobei Oxazolin ausgenommen ist,
sowie deren physiologisch verträglichen Salze, dadurch gekennzeichnet, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

2. Verfahren gemäß Anspruch 1, dadurch gekennzeichnet, daß
$R^1$ ($C_2$-$C_{11}$)-Alkanoyl, wie n-Decanoyl, Formyl, Acetyl, Pivaloyl, Isovaleryl oder Isobutyryl; substituiertes ($C_2$-$C_{11}$)Alkanoyl, wie 2-Hydroxypropionyl 2-Hydroxy-3-methyl-butyryl oder gegebenenfalls geschützes Amino-($C_2$-$C_{11}$)-alkanoyl, wie 4-Aminobutyryl, 5-Aminopentanoyl, 6-Aminohexanoyl, 4-N-tert.-Butoxycarbonylaminobutyryl, 5-N-tert.-Butoxycarbonylaminopentanoyl oder 6-N-tert.-Butoxycarbonylaminohexanoyl oder Di-($C_1$-$C_7$)-alkylamino-($C_2$-$C_{11}$)-alkanoyl, wie Dimethylaminoacetyl; ($C_4$-$C_9$)-Cycloalkylcarbonyl, wie Cyclopropylcarbonyl, Cyclobutylcarbonyl, Cyclopentylcarbonyl oder Cyclohexylcarbonyl; ($C_6$-$C_{10}$)-Aryl-($C_2$-$C_{11}$)-alkanoyl, wie Phenylacetyl, Phenylpropanoyl oder Phenylbutanoyl, 2-(2,6-Dichloroanilino)-phenylacetyl, 2-(N-Benzyl-2,6-dichloranilino)-phenylacetyl; gegebenenfalls durch Halogen, ($C_1$-$C_7$)-Alkyl, ($C_1$-$C_7$)-Alkoxy oder ($C_1$-$C_7$)-Alkoxycarbonyl substituiertes Benzoyl, wie 4-Chlorbenzoyl, 4-Methylbenzoyl, 2-Methoxycarbonylbenzoyl

oder 4-Methoxybenzoyl, Pyrrolyl-2-carbonyl, Pyridyl-3-carbonyl, Benzylsulfonyl; ($C_2$-$C_{11}$)-Alkoxycarbonyl, wie Methoxycarbonyl, Ethoxycarbonyl oder tert.-Butoxycarbonyl; durch Halogen substituiertes ($C_2$-$C_{11}$)-Alkoxycarbonyl, wie 2,2,2-Trichlorethoxycarbonyl oder 1,1-Dimethyl-2,2,2-trichlorethoxycarbonyl; ($C_6$-$C_{14}$)-Aryl-($C_2$-$C_7$)-alkoxycarbonyl, wie Benzyloxycarbonyl oder 9-Fluorenylmethylcarbonyl bedeutet.

3. Verfahren gemäß Anspruch 1 und/oder 2, dadurch gekennzeichnet, daß $R^2$ Isobutyl, Benzyl oder Cyclohexylmethyl bedeutet.

4. Verfahren gemäß einem oder mehreren der Ansprüche 1 bis 3, dadurch gekennzeichnet, daß $R^3$ Wasserstoff, Isopropyl oder Isobutyl bedeutet.

5. Verfahren gemäß einem oder mehreren der Ansprüche 1 bis 4, dadurch gekennzeichnet, daß A und B unabhängig voneinander Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, Lysin, Ornithin, 2,4-Diaminobuttersäure, Arginin, Norvalin, 4-Chlorphenylalanin, Methioninsulfon, Methioninsulfoxid, 2-Pyridylalanin, 3-Pyridylalanin, 4-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphtylalanin, 4-Nitrophenylalanin, Norleucin, Valin, Alanin, 1,2,3,4-Tetrahydroisochinolin-3-carbonsäure, Homophenylalanin, 2-Amino-4-(2-thienyl)-buttersäure, (Z)-Dehydrophenylalanin oder (E)-Dehydrophenylalanin bedeuten.

6. Verfahren gemäß einem oder mehreren der Ansprüche 1 bis 5, dadurch gekennzeichnet, daß $R^7$ für den Rest eines 5-gliedrigen Heterocyclus mit einem oder zwei Heteroatomen, der gegebenenfalls durch ($C_1$-$C_7$)-Alkyl substituiert ist, steht.

7. Verwendung einer nach einem der Verfahren der Ansprüche 1 bis 6 hergestellten Verbindung der Formel I als Heilmittel.

8. Verwendung einer nach einem der Verfahren der Ansprüche 1 bis 6 hergestellten Verbindung der Formel I bei der Behandlung des Bluthochdrucks.

9. Verfahren zur Herstellung einer pharmazeutischen Zübereitung enthaltend eine nach einem der Verfahren der Ansprüche 1 bis 6 hergestellten Verbindung der Formel I, dadurch gekennzeichnet, daß man diese zusammen mit einem physiologisch unbedenklichen Träger und gegebenenfalls weiteren Hilfs- und Zusatzstoffen in eine geeignete Darreichungsform bringt.

Europäisches Patentamt

# EUROPÄISCHER RECHERCHENBERICHT

Nummer der Anmeldung

EP 90 10 7395

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,Y | EP-A-0 255 082 (HOECHST)<br>* Ganzes Dokument, insbesondere die allgemeine Beschreibung, Seiten 2-4 *<br>--- | 1-9 | C 07 K 5/06<br>C 07 K 5/02<br>A 61 K 37/64 |
| Y | EP-A-0 231 919 (SQUIBB)<br>* Ganzes Dokument, insbesondere die Beschreibung; Seiten 1-7, Beispiele *<br>--- | 1-9 | |
| A | EP-A-0 310 015 (HOECHST)<br>* Das ganze Dokument *<br>--- | 1-9 | |
| A | EP-A-0 202 577 (SEARLE)<br>* Ganzes Dokument, insbesondere Beispiel 10, Seite 25 *<br>--- | 1-9 | |
| A | EP-A-0 292 800 (MERCK PATENT-GESELLSCHAFT)<br>* Ganzes Dokument, insbesondere Beispiel 7 *<br>--- | 1-9 | |
| A | DE-A-3 721 855 (MERCK PATENT-GESELLSCHAFT)<br>* Ganzes Dokument, insbesonder Beispiele 5,7 *<br>----- | 1-9 | **RECHERCHIERTE SACHGEBIETE (Int. Cl.5)**<br>C 07 K<br>A 61 K |

Der vorliegende Recherchenbericht wurde für alle Patentansprüche erstellt

| Recherchenort | Abschlußdatum der Recherche | Prüfer |
|---|---|---|
| DEN HAAG | 24-07-1990 | MASTURZO P. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Grunden angeführtes Dokument

& : Mitglied der gleichen Patentfamilie, übereinstimmendes Dokument

EPO FORM 1503 03.82 (P0403)